# EUROPÄISCHE PATENTSCHRIFT

(11) **EP 0 799 177 B1**
(45) Veröffentlichungstag und Bekanntmachung des Hinweises auf die Patenterteilung: **05.04.2000**
(21) Anmeldenummer: 95941709.8
(22) Anmeldetag: 13.12.1995
(51) Int. Cl.: C07C 51/41, C07C 59/68, C07C 59/70

(54) **VERFAHREN ZUR HERSTELLUNG FESTER, FREIFLIESSENDER WASSERLÖSLICHER SALZE VON ARYLOXI-C 1-C 4-ALKANCARBONSÄUREN**
PROCESS FOR PREPARING SOLID, FREE-FLOWING WATER-SOLUBLE SALTS OF ARYLOXY-C 1-C 4-ALKANE CARBOXYLIC ACIDS
PROCEDE DE PREPARATION DE SELS SOLIDES COULANTS SOLUBLES DANS L'EAU D'ACIDES ARYLOXY-ALCANE-C 1-C 4-CARBOXYLIQUES

(30) Priorität: 23.12.1994 DE 4446387
(43) Veröffentlichungstag der Anmeldung: 08.10.1997
(73) Patentinhaber: BASF AKTIENGESELLSCHAFT, 67056 Ludwigshafen (DE)
(72) Erfinder: MERKLE, Hans, Rupert, D-67061 Ludwigshafen (DE); BRENNER, Karl, Siegfried, D-67071 Ludwigshafen (DE); FRETSCHNER, Erich, D-69239 Neckarsteinach (DE); SCHÖNHERR, Michael, D-67227 Frankenthal (DE); VAN GASTEL, Anne, D-67435 Neustadt-Königsbach (DE)
(86) Internationale Anmeldenummer: EP9504934
(87) Internationale Veröffentlichungsnummer: WO9620155

(56) Entgegenhaltungen:
- DE-A- 2 520 262
- DE-A- 2 531 643
- DE-C- 883 606

## Beschreibung

Die vorliegende Erfindung betrifft ein Verfahren zur Herstellung fester, freifließender wasserlöslicher Salze von Aryloxi-C₁-C₄-alkancarbonsäuren.

Die Stoffklasse der Aryloxialkancarbonsäuren, insbesondere der Aryloxiessigsäuren, 2-Aryloxi-propansäuren und 4-Aryloxibutansäuren, wobei als Arylrest insbesondere ein durch Halogen wie Brom oder Chlor und/oder C₁-C₄-Alkyl, insbesondere Methyl substituierter Phenylrest zu verstehen ist, ist seit langem bekannt für ihre herbizide Wirkung. Eine Zusammenstellung findet man z.B. in K.H. Büchel: Pflanzenschutz und Schädlingsbekämpfung, Georg Thieme Verlag, Stuttgart 1977, S. 173-175. Kommerzielle Verwendung fanden z.B. die folgenden Wirkstoffe: 2,4-Dichlorphenyoxiessigsäure (2,4D), 2-Methyl-4-chlorphenoxyessigsäure (MCPA), 2-(2-Methyl-4-chlorphenoxi)propionsäure (Mecoprop, MCPP), 2-(2,4-Dichlorphenoxy)propionsäure(Dichlorprop, 2,4-DP), 2-(2,4,5-Trichlorphenoxi)propionsäure (Fenoprop), 4-(2,4-Dichlorphenoxi)butansäure (2,4DB) und 4-(2-Methyl-4-chlorphenoxi)butansäure (MCPB) und deren Salze, insbesondere von Natrium, Kalium, Ethylammonium und Dimethylammonium (vgl. auch The Pesticide Manual, 9th ed., published by the British Crop Protection, Council, 1991).

Die Vermarktung der Aryloxicarbonsäuren, nachfolgend auch als Phenoxicarbonsäuren bezeichnet, erfolgt überwiegend in Form ihrer Salze als flüssige Konzentrate. Wäßrige Lösungen von Phenoxicarbonsäure-Salzen sind z.B. aus US 2,694,625, US 2,992,913 und US 3,284,186 bekannt. Diese Salzlösungen werden in Kunststoff-bzw. Metallbehältern gehandhabt, deren Entsorgung bzw. Reinigung öffentliche Diskussionen hervorriefen.

Es bestand daher ein Bedarf, diese Verbindungen in fester und gleichzeitig gut wasserlöslicher Form zur Verfügung zu stellen.

Bereits 1950 wurden granulierte Formulierungen von halogenierten Phenoxiessigsäuren offengelegt, bei denen zur Vermeidung von Staubanteilen Hilfsstoffe wie Lehm, Kalk, Gips, Kunstdünger zugesetzt wurden (US 2,792,295).

Es wurden auch feste, freifließende, wasserlösliche Lithium-Salze von Phenoxicarbonsäuren beschrieben (US 3,208,843). US 3,023,096 lehrt die Herstellung der Kalium- und Lithiumsalze von Dichlorphenoxiessigsäure.

Im US-Patent 5,266,553 wird der Stand der Technik ausführlich diskutiert und zur Herstellung fester Salze vorgeschlagen, die entsprechenden Aryloxicarbonsäuren mit einer Base zu neutralisieren und die resultierende wäßrige Lösung bzw. Maische durch Verdampfen von Wasser unter kontrollierten Bedingungen in die festen Salze zu überführen. Ebenso sind durch Verdampfen von Wasser aus den entsprechenden kommerziell erwerbbaren Aryloxisalzlösungen die festen Salze herzustellen.

Aus der DE-A-2531643 ist die Herstellung von Natrium-α-[4-(4-trifluormethylphenoxy)-phenoxy]-propionat aus einer ethanolischen wäßrigen Lösung mit NaOH und anschließender Verdampfung des Lösungsmittels bekannt.

Veröffentlichungen über die Herstellung von festen, fließfähigen Salzen optisch aktiver Phenoxipropionsäuren sind bisher nicht bekannt.

Die optisch aktiven Phenoxipropionsäuren werden durch Reaktion der aus der Synthese gewonnenen neutralen Reaktionslösungen durch Ansäuren mit Säuren freigesetzt, isoliert und in die Salze übergeführt (DE-A 30 24 265).

Gemäß EP-A 0 009 285 wird aus der teilweise aufbereiteten neutralen Reaktionslösung mit Salzsäure bei pH 4 eine ölige Phase abgetrennt, welche mit Ether extrahiert wird. Nach Verdampfen des Ethers erhält man die entsprechenden optisch aktiven Phenoxipropionsäuren.

Der Erfindung lag nun die Aufgabe zugrunde, ein Verfahren zur Herstellung fester, frei fließender und gut wasserlöslicher Salze von Aryloxicarbonsäuren zur Verfügung zu stellen, welches sich insbesondere auch für die Herstellung optisch aktiver Propionate, von denen bekanntlich ausschließlich die D-(+)-Enantiomeren biologisch aktiv sind (vgl. K.H. Büchel, loc. cit. und Kgl. Lantbruks-Högskolans, Anm. 20, 241-295 (1953)), eignet.

Demgemäß wurde ein Verfahren zur Herstellung fester, freifließender, wasserlöslicher Salze von Aryloxi-C₁-C₄-alkancarbonsäuren gefunden, welches dadurch gekennzeichnet ist, daß man die Aryloxi-C₁-C₄-alkancarbonsäuren mit einer salzbildenden Base umsetzt, wobei die Salzbildung in der Schmelze in Abwesenheit eines Schleppmittels/eines Lösungsmittels erfolgt und dann die festen Salze in üblicher Weise isoliert.

Die Überführung der Säure in ihr Salz erfolgt mit den dafür üblichen Basen. Hier sind Alkali- und Erdalkalihydroxide, z.B. Natriumhydroxid, Kaliumhydroxid und Magnesiumhydroxid, Alkali- bzw. Erdalkalioxide, Alkali- bzw. Erdalkalicarbonate, Hydrogencarbonate, Alkali- bzw. Erdalkaliacetate und -formiate, Ammoniak und alkylsubstituierte primäre, sekundäre und tertiäre Amine, mit 1 bis 4 C-Atomen zu nennen oder Mischungen dieser Basen. Die Basen können stöchiometrisch im Überschuß oder schwachen Unterschuß eingesetzt werden.

Die Schmelzen werden direkt mit der Base durch innige Vermischung zur Reaktion gebracht.

Die auf den genannten Wegen hergestellten festen, fließfähigen Salze von Phenoxicarbonsäuren können in volumensparende Papier- oder Kunststoffsäcke abgepackt und gelagert werden. Vor der Anwendung können sie sehr leicht zu wäßrigen Spritzbrühen definierter Konzentrationen gelöst werden. Die Entsorgung dieser Gebinde ist einfach und mögliche Probleme mit von Produkt kontaminierten Kunststoff- oder Metallbehältern entfallen.

Nach einer weiteren erfindungsgemäßen Ausführungsform des Verfahrens können nicht nur einzelne Aryloxicarbonsäuresalze, sondern auch Mischungen von Salzen verschiedener Aryloxicarbonsäuren hergestellt werden. Beispielsweise seien folgende Mischungen aus zwei bzw. drei Phenoxicarbonsäuren aufgeführt:
(jeweils common name, vgl. The Pesticide Mannual, 9th edition, 1991)
Dichlorprop-P und 2,4-D;
Dichlorprop-P und MCPA;
Mecoprop-P und 2,4-D;
Mecoprop-P und MCPA;
MCPA und 2,4-D;
Mecoprop-P, Dichlorprop-P und MCPA.

Anstelle der optisch aktiven Phenoxicarbonsäuren können auch deren Racemate in der Mischung vorliegen. Weiterhin ist es möglich, neben den Phenoxicarbonsäuren als Ausgangsstoffe andere Herbizide, ausgewählt aus der Gruppe Bentazon, Glyphosate, Glufosinate, Ioxynil, Bromoxynil und Dicamba (vgl. The Pesticide Manual, loc. cit., Nr. 770, 6950, 6930, 7300, 1410 und 4100) vorliegen zu haben und aus deren Mischung direkt die entsprechenden, festen Salzgemische, insbesondere von Natrium-, Kalium- oder Ammoniumsalzen herzustellen. Beispielsweise seien folgende Mischungen aufgeführt:
Bentazon und 2,4 D;
Bentazon und Dichlorprop-P;
Bentazon und Mecoprop-P;
Bentazon und MCPA;
Bentazon und MCPB;
Bentazon und MCPB und MCPA;
Bentazon und 2,4 DB;
Bentazon und MCPA.

Bevorzugt werden aus diesen Mischungen nach dem erfindungsgemäßen Verfahren die Natrium- bzw. Kaliumsalze gebildet. Anstelle von Bentazon in o.g. Mischungen können in analoger Weise Glyphosate, Glufosinate, Ioxynil, Bromoxynil und Dicamba umgesetzt werden.

Die nachfolgenden Beispiele erläutern die Erfindung.

### Herstellungsbeispiele:

### Beispiel 1

### 2-Methyl-4-chlorphenoxiessigsäure-Kaliumsalz

In eine Schmelze von 20 Teilen (0,1 Mol) MCPA (93,3 %ig) werden unter Rühren 6,6 Teile (0,1 Mol) gepulvertes Kaliumhydroxid (85 %ig) eingetragen und 30 Minuten bei 130°C gehalten. Nach dem Erstarren erhält man 26 Teile MCPA-Kaliumsalz mit einem MCPA Gehalt von 71,8 %, was einer Ausbeute von 100 % entspricht. Fp. 203°C.

Die auf diesen Wegen hergestellten Salze bzw. Salzmischungen sind vollständig wasserlöslich.

### Beispiel 2

### D-2-Methyl-4-chlorphenoxipropionsäure-Kaliumsalz

In eine Schmelze von 21,4 Teile (0,1 mol) D-CMPP (D+L Gehalt 98,3 %; D: 97,4 % L: 2,6 %) werden bei 130°C 14 Teile (0,14 mol) Kaliumhydrogencarbonat eingetragen, wobei CO₂ freigesetzt wird. Im Wasserstrahlvakuum werden Restwassermengen entfernt. Man erhält 28,8 Teile D-CMPP-Kaliumsalz mit einem Säuregehalt von 72,0 %, bestehend aus 97,4 % D und 2,6 % L Enantiomer - vom Fp. 190°C-195°C, was einer Ausbeute von 95,3 % entspricht.

## Patentansprüche

1. Verfahren zur Herstellung fester, freifließender wasserlöslicher Salze von Aryloxi-C₁-C₄-alkancarbonsäuren, dadurch gekennzeichnet, daß man die Aryloxi-C₁-C₄-alkancarbonsäuren mit einer salzbildenden Base umsetzt, wobei die Salzbildung in der Schmelze in Abwesenheit eines Schleppmittels/eines Lösungsmittels erfolgt und dann die festen Salze in üblicher Weise isoliert werden.

2. Verfahren nach Anspruch 1, dadurch gekennzeichnet, daß man als salzbildende Base Hydroxide, Oxide, Carbonate, Acetate oder Formiate von Alkali- oder Erdalkalimetallen; Ammoniak oder C₁-C₄-Alkylamine verwendet.

3. Verfahren nach den Ansprüchen 1 oder 2, dadurch gekennzeichnet, daß man von optisch aktiven 2-Aryloxipropionsäuren oder deren Salzen ausgeht und diese unter Erhalt der optischen Aktivität in die festen wasserlöslichen Salze überführt.

4. Verfahren nach den Ansprüchen 1 bis 3, dadurch gekennzeichnet, daß man eine Mischung von Salzen verschiedener Aryloxi-C₁-C₄-alkancarbonsäuren herstellt.

5. Verfahren nach Anspruch 4, dadurch gekennzeichnet, daß man feste, freifließende Salze folgender Mischungen herstellt:
Dichlorprop-P und 2,4-D;
Dichlorprop-P und MCPA;
Mecoprop-P und 2,4-D;
Mecoprop-P und MCPA;
MCPA und 2,4-D;
Mecoprop-P, Dichlorprop-P und MCPA.

6. Verfahren nach den Ansprüchen 1 bis 5, dadurch gekennzeichnet, daß man die Salze von Aryloxi-C₁-C₄-alkancarbonsäuren im Gemisch mit den entsprechenden Salzen anderer Herbizide, ausgewählt aus der Gruppe Bentazon, Glyphosate, Glufosinate, Ioxynil, Bromoxynil und Dicamba herstellt.

## Claims

1. A process for preparing solid, free-flowing water-soluble salts of aryloxy-C₁-C₄-alkanecarboxylic acids, which comprises reacting the aryloxy-C₁-C₄-alkanecarboxylic acids with a salt-forming base, the salt formation taking place in the melt in the absence of an entraining agent/a solvent and the solid salts then being isolated in a customary manner.

2. A process as claimed in claim 1, wherein the salt-forming base used is a hydroxide, oxide, carbonate, acetate or formate of alkali metals or alkaline earth metals; ammonia or C₁-C₄-alkylamines.

3. A process as claimed in claims 1 or 2, wherein optically active 2-aryloxypropionic acids or their salts are used as starting materials and these are converted into the solid water-soluble salts with retention of the optical activity.

4. A process as claimed in claims 1 to 3, wherein a mixture of salts of various aryloxy-C₁-C₄-alkanecarboxylic acids is prepared.

5. A process as claimed in claim 4, wherein solid, free-flowing salts of the following mixtures are prepared:
dichlorprop-P and 2,4-D;
dichlorprop-P and MCPA;
mecoprop-P and 2,4-D;
mecoprop-P and MCPA;
MCPA and 2,4-D;
mecoprop-P, dichlorprop-P and MCPA.

6. A process as claimed in claims 1 to 5, wherein the salts of aryloxy-C₁-C₄-alkanecarboxylic acids are prepared in a mixture with the corresponding salts of other herbicides, selected from the group consisting of bentazone, glyphosate, glufosinate, ioxynil, bromoxynil and dicamba.

## Revendications

1. Procédé pour préparer des sels d'acides aryloxy-(alcane en C1-C4)carboxyliques solides, s'écoulant librement et solubles dans l'eau, caractérisé par le fait que l'on fait réagir les acides aryloxy-(alcane en C1-C4)carboxyliques avec une base salifiante, la salification étant réalisée à l'état fondu en l'absence d'un agent d'entraînement et d'un solvant, les sels solides étant ensuite isolés de la manière habituelle.

2. Procédé selon revendication 1, caractérisé par le fait que l'on utilise en tant que bases salifiantes des hydroxydes, des oxydes, des carbonates, des acétates ou des formiates de métaux alcalins ou alcalino-terreux ; l'ammoniac ou des alkylamines en C1-C4.

3. Procédé selon les revendications 1 ou 2, caractérisé par le fait que l'on part d'acides 2-aryloxypropioniques à l'état d'isomères optiques ou de leurs sels et qu'on les convertit en les sels solides solubles dans l'eau avec conservation de l'activité optique.

4. Procédé selon les revendications 1 à 3, caractérisé par le fait que l'on prépare un mélange de sels de plusieurs acides aryloxy-(alcane en C1-C4)carboxyliques.

5. Procédé selon revendication 4, caractérisé par le fait que l'on prépare des sels solides s'écoulant librement des mélanges suivants :
Dichlorprop-P et 2,4-D ;
Dichlorprop-P et MCPA ;
Mecoprop-P et 2,4-D ;
Mecoprop-P et MCPA ;
MCPA et 2,4-D ;
Mecoprop-P, Dichlorprop-P et MCPA.

6. Procédé selon les revendications 1 à 5, caractérisé par le fait que l'on prépare les sels des acides aryloxy-(alcane en C1-C4)carboxyliques en mélange avec les sels correspondants d'autres herbicides choisis dans le groupe consistant en les suivants : Bentazon, Glyphosate, Glufosinate, Ioxynil, Bromoxynil et Dicamba.
